# EUROPEAN PATENT APPLICATION

(11) **EP 3 267 213 A1**
(43) Date of publication of application: **10.01.2018**
(21) Application number: 16178580.3
(22) Date of filing: 08.07.2016
(51) Int. Cl.: G01R 33/022, A61B 5/04, A61B 5/00

(54) **SENSOR FOR MEASURING MAGNETIC FIELDS**

(71) Applicant: Biomagnetik Park GmbH, 21149 Hamburg (DE)
(72) Inventor: KIM, Byeongsoo, 22455 Hamburg (DE); KIM, Bonggun, 20535 Hamburg (DE)
(74) Representative: Stüven, Ralf

(57) **Abstract**

The invention relates to a sensor for magnetic field measuring apparatus and a magnetic field measuring apparatus. It is an object of the invention to provide an improved magnetic field sensor, in particular a reliable magnetic field sensor which can comparatively easy be manufactured and is comparatively robust in terms of e.g. wiring connections. For solving the problem, the invention provides, in a first aspect, a magnetic field sensor (1) for a magnetic field measuring apparatus, in particular a biomagnetic field measuring apparatus, comprising a sensor bobbin (2) and at least one superconducting quantum interference device (3) comprising a detection coil (4) of superconducting wiring (5), the sensor bobbin (2) comprising grooves (6, 7, 12) and holes (8) formed in the wall (9) of the sensor bobbin (2) as guide means for guiding at least part of the superconducting wiring (5) of the detection coil (4).

## Description

The invention relates to a sensor for measuring magnetic fields, in particular weak magnetic fields like biomagnetic fields, and a magnetic field measuring apparatus.

Magnetic field measuring sensors are generally known, and are widely used e.g. for measuring the Earth's magnetic field (geomagnetic field), magnetic fields generated by living organisms or tissues (biomagnetic fields), or as metal detetors. Especially when it comes to measuring weak magnetic fields like biomagnetic fields superconductive quantum interferometer devices (SQUIDs) are often used.

Examples for apparatus comprising magnetic field measuring sensors for measuring weak magnetic fields are biomagnetic field measuring apparatus like magnetocardiographs and magnetoencephalographs, measuring very weak magnetic fields generated by muscle or nerve tissue, e.g. the heart or brain. Biomagnetic field mesuring apparatus are e.g. described in US 5,113,136, US 5,644,229, US 6,230,037 B1, US 6,424 853 B1, US 6,842,637 B2, or US 7,194,121 B2. Magnetocardiography (MCG) and Magnetoencephalography (MEG) are established non-invasive methods used e.g. for examining subjects for abnormal conditions or diseases of the heart or brain.

For measuring weak magnetic fields, for example the very weak biomagnetic fields generated by tissue of living organisms, e.g. by heart muscle or brain tissue, sensors have to be used, which are very sensitive and consequently also prone to interference from external magnetic fields. The design of magnetic field sensors is therefore particularly important for apparatus measuring weak magnetic fields, e.g. for biomagnetic field measuring apparatus.

The design of magnetic field sensors known from the prior art still needs improvement. US 6,424 853 B1, for example, describes vector magnetometers comprising detection coils for detecting magnetic fields of three directions and a superconductive quantum interferometer device (SQUID) connected to the detection coils. The production of these magnetometers is, however, comparatively expensive, and wire connections are prone to breakage.

US 5049818 describes a superconducting gradiometer for measuring weak magnetic fields for a measuring device comprising a SQUID. A coil of the gradiometer made of superconducting wire is arranged about a carrier body in axial and circumferential grooves cut in the carrier body, the grooves being coated with a superconducting thin film. The superconducting wire is fixed in the grooves using an adhesive.

US 20030141868 A1 describes a gradiometer comprising a non-magnetic insulating gradiometer support having a first coefficient of thermal expansion. The support comprises near horizontal near circular grooves connecting straight near vertical grooves. The superconducting wire of the gradiometer has a second coefficient of thermal expansion and is retained inside the grooves using glue.

It is an object of the invention to provide an improved magnetic field sensor, e.g. biomagnetic field sensor, in particular a reliable magnetic field sensor for weak magnetic fields, e.g. biomagnetic fields, which can comparatively easy be manufactured and is comparatively robust in terms of e.g. wiring connections.

For solving the problem, the invention provides, in a first aspect, a magnetic field sensor for a magnetic field measuring apparatus, in particular a biomagnetic field measuring apparatus, comprising a sensor bobbin and at least one superconducting quantum interference device comprising a detection coil of superconducting wiring, the sensor bobbin comprising grooves and holes formed in the wall of the sensor bobbin as guide means for guiding at least part of the superconducting wiring of the detection coil.

The invention provides an improved magnetic field sensor. It solves, for example, the problem of disrupting or disconnecting superconducting wiring of magnetometers or gradiometers. In prior art sensors, wire connections immersed in e.g. liquid helium in a sensor bobbin tend to be easily broken due to mechanical stress. The invention solves this problem by providing grooves and holes as guide means for guiding the superconducting wiring of the magnetometer(s) or gradiometer(s). The guide means not only hold the wires tightly without any adhesive material, but also serve the purpose of forming coils having a well-defined area.

The term "weak magnetic field" relates to magnetic fields below 1 mT. Examples for weak magnetic fields are the geomagnetic field or biomagnetic fields.

The term "biomagnetic field" relates to magnetic fields generated by electric currents in cells, tissue or organs, e.g. heart or brain tissue.

The term "magnetic field sensor" as used herein means a sensor being able to measure a (bio)magnetic field.

The terms "SQUID" or "superconducting quantum interference devices" (see e.g. Fagaly, R.L., 2006, Superconducting quantum interference device instruments and applications, Rev. Sci. Instrum. 77, 101101, doi: 10.1 063/ 1.235 4545) relates to a very sensitive magnetic field measuring device based on superconducting loops containing Josephson junctions used to measure weak magnetic fields. SQUIDs comprise a detection coil connected to it with superconducting wires (e.g. of niobium), allowing current from the detection coils to inductively couple to the SQUID.

The term "detection coil" as used herein relates to coils of superconducting material connected to SQUIDs. Detection coils may have different geometries, and e.g. be configured as simple "magnetometers" with only one coil ("pickup coil"), or as first or second derivative gradiometers having two or more coils, which may be placed axially above each other (axial gradiometer). The term "flux transformer" may also be used synonymous for the term "detection coil".

The term "magnetometer" as used herein relates to detection coils having only a single coil (pickup coil, with one or several turns in the same direction.

The term "gradiometer" as used herein relates to detection coils having two or more coils (with one or more turns). A first derivative (also "first order") gradiometer has e.g. two oppositely wound coils (pickup coil and compensation coil), spaced apart, and placed, for example, axially above each other (axial gradiometer). Second derivative (or "second order") gradiometers have e.g. three axially spaced apart coils, a pickup coil and two further coils. The direction and number of turns of the three coils may have the relation +n : -2n : +n (e.g. one turn for the pickup coil, two turns for the second coil in the opposite direction and 1 turn for the third coil in the direction of the pickup coil).

The term "pickup coil" as used herein relates to a coil (having one or more turns) of a detection coil placed near the source of the magnetic field.

The term "input coil" or "coupling coil" as used herein relates to a coil of the detection coil for inductively coupling current from the detection coil to a SQUID.

The terms "1-axis magnetic field sensor", "2-axis magnetic field sensor" or "3-axis magnetic field sensor" refer to magnetic field sensors measuring only one, two or three of the three orthogonal components (x, y, z) of the magnetic field, i.e. the. A "3-axis magnetic field sensor" is e.g. a magnetic field sensor measuring the components of the magnetic field in all three dimensions. The term "2-axis magnetic field sensor" encompasses sensors being composed of at least two magnetometers or gradiometers measuring the orthogonal x- and y-, x- and z- or y-and z-components of a magnetic field. Likewise, the term "3-axis magnetic field sensor" encompasses sensors being composed of at least three magnetometers or gradiometers measuring the orthogonal x-, y-, and z-components of a magnetic field.

The terms "first component", "second component" or "third component" in relation to a magnetic field refer to the orthogonal components of a magnetic field. Instead, also the terms "x-component" (for e.g. the first component), "y-component" (for e.g. the second component) and "z-component" (for e.g. the third component may be used. The terms refer to the components of any set of orthogonal magnetic field components, without being restricted to a specific meaning of the terms in relation to e.g. a plane or axis of, for example, a human body. In particular, the terms "x-component" and "y-component" preferably refer to the components of the magnetic field in direction of the x- and y-axis, respectively, of a plane (x-y plane) formed by or parallel to a body surface, e.g. the front or back of a human thorax, or the surface of the cranium. The term "z-component" preferably relates in particular to the component in direction of the z-axis, i.e perpendicular to the x-y plane. A reference to an x-axis when measuring magnetic fields of the heart of a human being preferably corresponds to a reference to a right-to-left axis, a reference to an y-axis preferably corresponds to a reference to a head-to-foot axis, and a reference to the z-axis preferably corresponds to a reference to a anteroposterior axis, wherein "right", "left", "head", "foot", and "anteroposterior" relate to the body of a human being.

The term "subject" as used herein refers preferably to a vertebrate, further preferred to a mammal, and most preferred to a human.

The expression according to which a detection coil is designed and arranged to measure a specific component, i.e. the first, second or third component (x-, y- or z-component) of a magnetic field means that the detection coil is constructed and/or oriented in a manner that only the respective component of the magnetic field is measured.

The grooves and holes are formed in the wall of the sensor bobbin. The grooves can, for example, be formed by indentations in the sensor bobbin wall. The superconducting wires forming at least part of the magnetometer(s) or gradiometer(s) can be inserted in and run along the grooves. The grooves may be provided in or on the outside or inside of the sensor bobbin wall. The grooves may may have a u-shape or v-shape in cross-section. Holes in the bobbin wall serve the purpose of allowing the wiring to enter or exit, as the case may be, the sensor bobbin. It is therefore preferred to position the holes in or at least in the vicinity of the grooves. The bobbin is preferably equipped with first grooves running in one direction, preferably in a direction pointing to the source of the magnetic field, and second grooves running perpendicular to the first grooves.

The grooves and holes provide for a means for guiding the superconducting wiring of the detection coil, and are designed and arranged in a manner that not only the superconducting wiring of the detection coil is secured but also coils can be formed having a well-defined area. The grooves and holes are, for example, designed and arranged in such a manner that a first-derivative gradiometer with a pickup coil and a compensation coil having the same area can be formed by simply placing the wiring in the grooves and by passing it through respective holes, if necessary. The holes are preferably formed within the grooves, e.g. in the grooves running in a direction pointing to the source of the magnetic field, and spaced apart in a suitable manner in order to enable e.g. the formation of coils being disposed axially above each other and having essentially the same area.

It is preferred, that the magnetic field sensor of the invention comprises two SQUIDs with corresponding detection coils each detecting a different component of the magnetic field. Consequently, the grooves and holes of the magnetic field sensor of the invention are preferably designed and arranged in such a number and manner that the superconducting wiring of two SQUIDs and corresponding detection coils for detecting different magnetic field components can be constructed with a single sensor bobbin. Further, the grooves and holes of the magnetic field sensor of the invention are preferably designed and arranged in such a number and manner that, with a single bobbin design, different combinations of detection coils can be realised, e.g. a combination of two detection coils measuring the x- and z-component of a magnetic field, a combination of two detection coils measuring the x- and y-component of a magnetic field, and a combination of two detection coils measuring the z- and y-component of a magnetic field.

In a preferred embodiment of the invention the sensor bobbin of the magnetic field sensor has an essentially tubular shape. The sensor bobbin may have an essentially rectangular, square, polygonal, elliptical or circular cross-section. In an especially preferred embodiment of the invention the sensor bobbin has a circular cross-section, i.e. has an essentially cylindrical shape.

In case of the sensor bobbin heaving an essentially tubular shape it is especially preferred that the sensor bobbin comprises longitudinal grooves, i.e. grooves running parallel to the longitudinal axis of the sensor bobbin, and transverse grooves, i.e. grooves running transversely to the longitudinal axis around the perimeter of the wall of the sensor bobbin, and holes placed in the longitudinal grooves and/or in the vicinity of the longitudinal grooves. In a particular preferred embodiment, the sensor bobbin also comprises bottom grooves formed in the bottom of the sensor bobbin.

In a preferred embodiment the longitudinal grooves are spread evenly over the perimeter of the wall of the sensor bobbin. The term "spread evenly over the perimeter of the wall of the sensor bobbin" means that the longitudinal grooves have essentially the same spacing from each other. In a further preferred embodiment of the invention the magnetic fied sensor comprises a) an even number of longitudinal grooves spread evenly over the perimeter of the wall of the sensor bobbin, such that for any of the longitudinal grooves a respective longitudinal groove is arranged on the opposite side of the bobbin wall, b) bottom grooves interconnecting longitudinal grooves on opposite sides of the sensor bobbin, c) at least two transverse grooves crossing the longitudinal grooves, and d) holes placed in the longitudinal grooves and/or in the vicinity of the longitudinal grooves.

In a preferred embodiment of the invention, the magnetic field sensor comprises at least one gradiometer-type detection coil having a pickup coil and a compensation coil, and grooves and holes for guiding the superconducting wiring of the at least one gradiometer-type detection coil leading to and forming the pickup coil and the compensation coil, such that the areas of the pickup coil and the compensation coil of the at least one gradiometer are essentially equal.

In a further preferred embodiment the magnetic field sensor of the invention comprises two gradiometer-type detection coils designed and arranged for measuring two different components of a magnetic field, the two different components of a magnetic field being orthogonal to each other, and comprises grooves and holes for guiding the superconducting wiring of the two gradiometer-type detection coils.

In a second aspect the invention also relates to a magnetic field measuring apparatus, in particular a biomagnetic field measuring apparatus, the magnetic field measuring apparatus comprising a plurality of magnetic field sensors according to the first aspect of the invention.

The magnetic field measuring apparatus comprises any suitable number of magnetic field sensors, for example 32, 64, 128, 256 or more magnetic field sensors according to the first aspect of the invention. This design is especially useful for biomagnetic field measuring apparatus, in particular magnetocardigraphs. The magnetic field measuring apparatus is, however, not limited to a specific number of magnetic field sensors.

In a preferred embodiment of the magnetic field measuring apparatus, the plurality of magnetic field sensors each comprise two gradiometer-type detection coils measuring two orthogonal components of a magnetic field, and comprise at least two, preferably three groups of magnetic field sensors differing in the combination of magnetic field components measured. For example, the first group of magnetic field sensors may be equipped with detection coils measuring a first and second component of a magnetic field, for example a biomagnetic field, e.g. the x- and y-component, the second group of magnetic field sensors may may be equipped with detection coils measuring the second and third component (y- and z-component) of the magnetic field, and the third group of magnetic field sensors may be equipped with detection coils measuring the third and first component (z- and x-component) of the magnetic field. This embodiment of the magnetic field measuring apparatus of the invention is designed to measure three orthogonal components of a magnetic field with a plurality of magnetic field sensors, although each of the magnetic field sensors is equipped with detection coils measuring only two orthogonal components of the magnetic field.

The magnetic field measuring apparatus preferably is a biomagnetic field measuring apparatus, for example a magnetoencephalograph (MEG) or magnetocardiograph (MCG), especially preferred a magnetocardiograph.

In the following, the invention is described in more detail by way of an example and the attached figures for illustration purposes only.
Fig. 1. Schematic illustration of an embodiment of a magnetic field sensor according to the invention in a spatial exterior view.
Fig. 2. Schematic illustration of a magnetic field sensor according to an embodiment of the invention in a front view (A), side view (B) and bottom view (C).
Fig. 3 Schematic illustrationof the embodiment of a magnetic field sensor according to the invention shown in Fig. 1 in another spatial view.
Fig. 4. Schematic illustration of detection coils types. a) magnetometer, b) first-derivative gradiometer.
Fig. 5 Schematic illustration of a detection coil (first-derivative gradiometer) inductively coupling to a SQUID.
Fig. 6 Schematic illustration of a part of a sensor bobbin comprising two detection coils.
Fig. 7 Schematic illustration of the embodiment of the sensor shown in Fig. 1 and 3 illustrating paths of the superconducting wiring. A. Sensor without superconducting wiring. B-D. Sensor with wiring for measuring an x-component (B), y-component (C) or z-component (D) of a magnetic field.

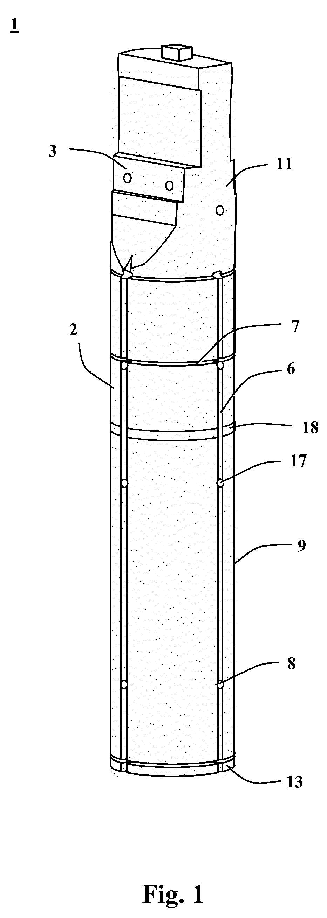

Figure 1 shows an embodiment of a magnetic field sensor 1 of the invention in a spatial exterior view. The magnetic field sensor 1 comprises a hollow cylindrical sensor bobbin 2 having a bobbin wall 9. In this embodiment, four longitudinal grooves 6, running parallel to the longitudinal axis 10 of the sensor bobbin 2, and three transverse (circumferential) grooves 7, running around the perimeter of the bobbin wall 9 and crossing the longitudinal grooves 6, are formed by indentations of the bobbin wall 9. The grooves 6, 7 may have a generally u- or v-shape in cross-section. Holes 8 in the bobbin wall 9 are provided in the longitudinal grooves 6. The sensor head 11 comprises two superconducting interference devices 3 (not directly visible here). In this embodiment, a hole 17 and a guide structure 18 serve for assembling the sensor 1 to a holding structure, not shown here.

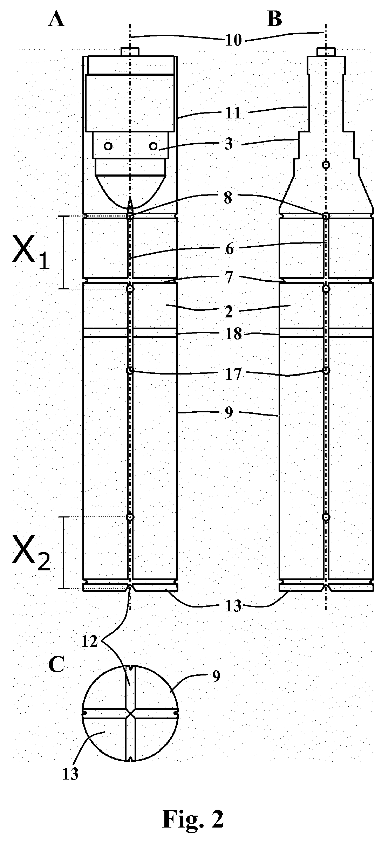

Figure 2 shows the magnetic field sensor 1 of figure 1 in a front view (A), a side view (B) and a bottom view (C). The bottom view (C) shows two orthogonal bottom grooves 12 connected with and extending the longitudinal grooves 6 along the bottom 13 of the bobbin 2. Each of the bottom grooves 12 interconnects two longitudinal grooves 6 on opposite sides of the sensor bobbin 2. As schematically indicated in Fig. 2, in this embodiment the spacing X₂ between the lower holes 8 and the bottom grooves 12 is the same as the spacing X₁ between two upper holes 8 near the sensor head 11. For example, by using the lower holes 8 and the bottom grooves 12 for forming a pickup coil 14 and the upper holes 8 for forming a corresponding compensation coil 15 of e.g. a first-derivative gradiometer, identical areas for the pickup coil 14 and the compensation coil 15 can easily be realized.

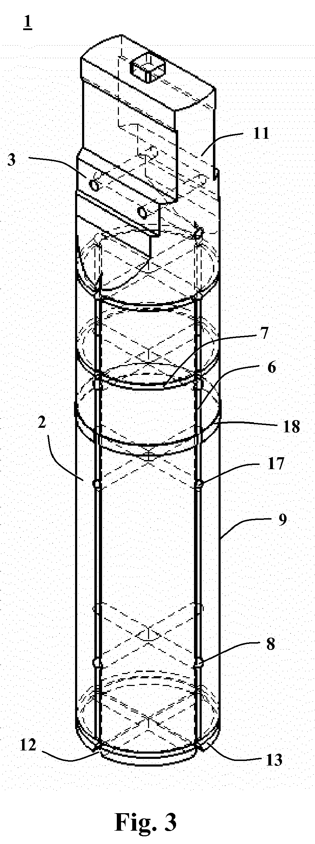

Figur 3 shows the embodiment of the magnetic field sensor 1 of figure 1 in another spatial view additionally showing inner structures of the sensor 1.

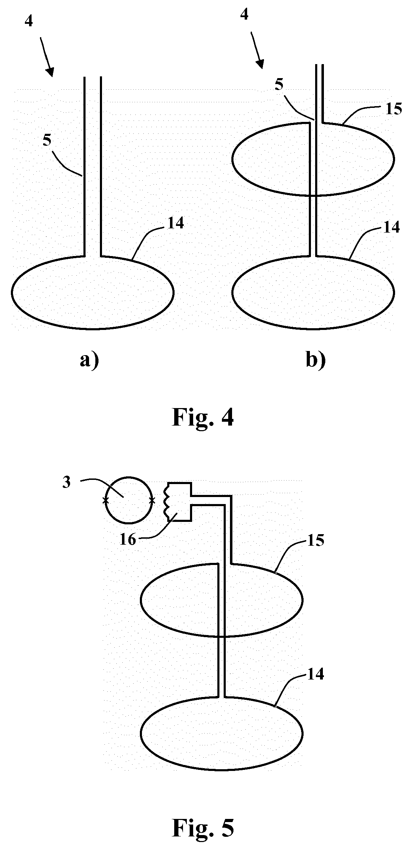

Figure 4 schematically illustrates two common types of detection coils 4. Figure 4a shows a magnetometer type detection coil 4 comprised of a pickup coil 14 and superconducting wiring leading to the pickup coil 14. Although not depicted here, the pickup coil 14 could have two or more turns. Figure 4b shows a gradiometer type detection coil, the gradiometer being a first-derivative gradiometer having a pickup coil 14 in the compensation coil 15. Although in this figure only one turn of the pickup coil 14 and compensation coil 15 are depicted, both could have two or more turns (e.g. two turns each).

Figure 5 schematically shows a first-derivative gradiometer type detection coil 4 of the superconducting wiring 5 including an input coil 16 inductively coupling to a SQUID 3.

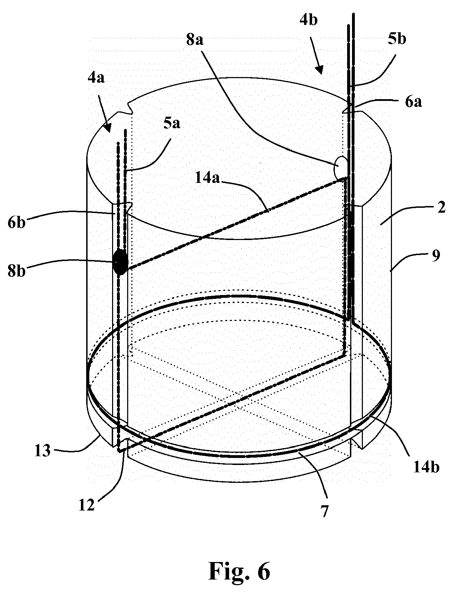

Figure 6 shows a detail of the bottom part of the sensor bobbin 2 according to the embodiment of the magnetic field sensor 1 shown in figure 1 and 2. The magnetic field sensor 1 comprises two different first-derivative gradiometer type detection coils 4a, 4b. For reasons of better overview only the pickup coils 14a, 14b of the detection coils 4a, 4b with part of the respective superconducting wiring 5a, 5b are shown. Part of the superconducting wiring 5a of the first gradiometer-type detection coil 4a is arranged in longitudinal grooves 6 on opposite sides of the sensor bobbin 2 and a bottom groove 12 connecting the opposite longitudinal grooves 6a, 6b. The superconducting wiring 5a enters the sensor bobbin 2 through a hole 8a placed in one of the longitudinal grooves 6a, extends through the interior of the sensor bobbin 2 and exits the sensor bobbin 2 through a hole 8b on the opposite side of the sensor bobbin 2. It is to be noted here that the dimensions of the holes 8 are adapted to the dimensions of the superconducting wiring 5. The holes 8 thus are as small as possible and do preferably not much exceed the diameter of the superconducting wiring 5 used in order to preferably only allow the necessary amount of superconducting wiring 5 to pass through the holes 8. The superconducting wiring 5a of the first detection coil 4a thus forms a rectangular pickup coil 14a having an area predefined by the dimensions of the sensor bobbin 2 and the position of the holes 8a, 8b. The superconducting wiring 5a leading to the compensation coil 15 (not shown here) comes to lie in the same groove 8b. The first detection coil 4a measures the x- or y-component of a magnetic field. The superconducting wiring 5b of the second gradiometer-type detection coil 4b is arranged in a longitudinal groove 6a and a transverse groove 7 around the perimeter of the sensor bobbin 2. The pickup coil 14b thus formed has a circular shape and also a well-defined area by virtue of the dimensions of the sensor bobbin 2. It is not necessary for the superconducting wiring 5b of the second detection coil 4b to enter or exit the sensor bobbin 2. The second detection coil 4b measures the z-component of the magnetic field.

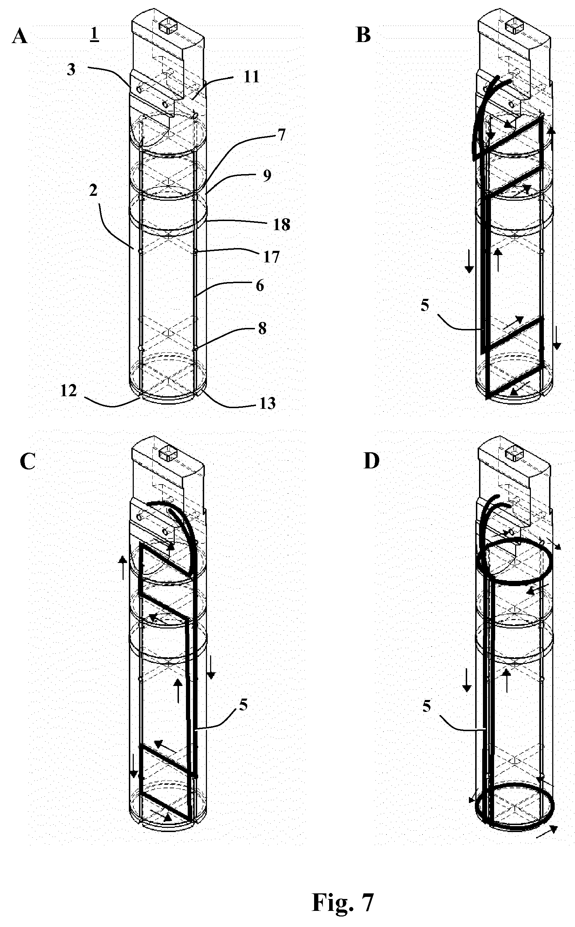

Figure 7 shows schematically and very roughly the approximate paths of the superconducting wiring 5. For reasons of better overview, the sensor 1 depicted in Fig. 3 is shown again in Fig. 7A. Reference signs are only given for Fig. 7A, and omitted in Fig. 7B-D. Fig. 7B shows the sensor 1 with superconducting wiring 5 suitable for measuring an x-component, Fig. 7C with superconducting wiring 5 suitable for measuring an y-component, and Fig. 7D with superconducting wiring 5 suitable for measuring a z-component of a magnetic field.

## Claims

1. Magnetic field sensor (1) for a magnetic field measuring apparatus, in particular a biomagnetic field measuring apparatus, comprising a sensor bobbin (2) and at least one superconducting quantum interference device (3) comprising a detection coil (4) of superconducting wiring (5), the sensor bobbin (2) comprising grooves (6, 7, 12) and holes (8) formed in the wall (9) of the sensor bobbin (2) as guide means for guiding at least part of the superconducting wiring (5) of the detection coil (4).

2. Magnetic field sensor according to claim 1, wherein the sensor bobbin (2) has an essentially tubular shape.

3. Magnetic field sensor according to claim 2, wherein the sensor bobbin (2) has an essentially rectangular, square, polygonal, elliptical or circular cross-section, preferably a circular cross-section.

4. Magnetic field sensor according to one of claims 1 to 3, wherein the sensor bobbin (2) comprises longitudinal grooves (6) running parallel to the longitudinal axis (10) of the sensor bobbin (2), transverse grooves (7) running around the perimeter of the wall (9) of the sensor bobbin (2), bottom grooves (12) connected with and extending the longitudinal grooves 6 along the bottom (13) of the sensor bobbin (2).

5. Magnetic field sensor according to claim 4, comprising a) an even number of longitudinal grooves (6) spread evenly over the perimeter of the wall (9) of the sensor bobbin (2), such that for any of the longitudinal grooves (6) a respective longitudinal groove (6) is arranged on the opposite side of the bobbin wall (9), b) bottom grooves (12) interconnecting longitudinal grooves (6) on opposite sides of the sensor bobbin (2), c) at least two transverse grooves (7) crossing the longitudinal grooves (6), and d) holes (8) placed in the longitudinal grooves (6) and/or in the vicinity of the longitudinal grooves (6).

6. Magnetic field sensor according to one of the previous claims, comprising at least one gradiometer-type detection coil (4) having a pickup coil (14) and a compensation coil (15), and grooves (6, 7, 12) and holes (8) for guiding the superconducting wiring (5) of the at least one gradiometer-type detection coil (4) leading to and forming the pickup coil (14) and the compensation coil (15), such that the areas of the pickup coil (14) and the compensation coil (15) of the at least one detection coil (4) are essentially equal.

7. Magnetic field sensor according to claim 6, comprising two gradiometer-type detection coils (4a, 4b), and comprising grooves (6, 7, 12) and holes (8) for guiding the superconducting wiring (5) of the two gradiometer-type detection coils (4a, 4b), the two gradiometer-type detection coils (4a, 4b) being designed and arranged to measure two different orthogonal components of the magnetic field.

8. A magnetic field measuring apparatus, in particular biomagnetic field measuring apparatus, comprising a plurality of magnetic field sensors (1) according to one of claims 1 to 7.

9. The magnetic field measuring apparatus according to claim 8, comprising at least 32, preferably at least 64, 128, 256 or more magnetic field sensors (1) according to one of claims 1 to 7.

10. The magnetic field measuring apparatus according to claim 8 or 9, the plurality of magnetic field sensors (1) each comprising two gradiometer-type detection coils (4a, 4b) measuring two orthogonal components of a magnetic field, and comprising three groups of magnetic field sensors (1) differing in the combination of magnetic field components measured.

11. The magnetic field measuring apparatus according to one of claims 8 to 10, wherein the magnetic field measuring apparatus is a biomagnetic field measuring apparatus, preferably a magnetoencephalograph or magnetocardiograph, especially preferred a magnetocardiograph.
